# EUROPEAN PATENT APPLICATION

(11) **EP 2 085 069 A1**
(43) Date of publication of application: **05.08.2009**
(21) Application number: 08101032.4
(22) Date of filing: 29.01.2008
(51) Int. Cl.: A61K 6/083

(54) **Dental composite material**

(71) Applicant: Coltene Whaledent AG, 9450 Altstaetten (CH)
(72) Inventor: Benz, Silvan, 9463, Oberriet (CH); Böhner, Ralf, 9451, Kriessern (CH)
(74) Representative: Hepp, Dieter

(57) **Abstract**

The invention concerns hardenable dental composite materials, comprising i) non-agglomerated nanofillers having particle sizes of about 1 to about 50 nm; and ii) core-shell polymer particles with an elastic core compound, preferably having a modulus of elasticity of less than about 18 MPa, more preferably of less than 14 MPa, most preferably of less than about 10 MPa; and a substantially non-elastic shell compound. The materials have improved properties that allow for advantageous restoration of molar and premolar teeth with the bulk filling technique.

## Description

The present invention relates to the technical field of dental restoration, in particular to dental composite materials for bulk fillings.

In principle, hardening of a dental composite material by polymerization necessarily results in a volume shrinkage. However, extensive volume shrinkage cannot be tolerated with dental fillings since the marginal seal with the tooth has to be assured. Thus, in order to counteract the volume shrinkage of polymerizable dental composite materials upon hardening by polymerization, filling materials are routinely incorporated (such as e.g. dental glass or pyrogenic silicic acid). Such filling materials do not shrink and thus reduce the overall volume shrinkage of the dental composite material.

A typical approach of reducing shrinkage in polymerizable dental of composite materials is set forth in US 2006/0252845 A1; various other approaches of reducing polymerization shrinkage are discussed therein (e.g., shrinkage forces can be reduced by an incremental filling technique, which method is however time-consuming and uncomfortable for both the patient and the dental practitioner).

However, these (highly) filled dental composite materials have been found to be not ideally suitable at least for restorations of molar and premolar teeth.

It is thus an object of the present invention to overcome the above-mentioned drawbacks, especially to provide a dental composite material that is more suitable for dental restoration of molar and premolar teeth.

A hardenable dental composite material according to the invention comprises:
i) non-agglomerated nanofillers having particle sizes of about 1 to about 50 nm;
ii) core-shell polymer particles with
   - an elastic core compound, preferably having a modulus of elasticity of less than about 18 MPa, more preferably of less than 14 MPa, most prefera-bly of less than about 10 MPa; and
   - a substantially non-elastic shell compound.

It has been found that the minor performance of prior art composite materials in molar and premolar teeth was essentially based on an inappropriate high modulus of elasticity. Thereby, the high force exposure during chewing directly impacted the marginal seal to the (adhesive layer and the) tooth. The presence of fillers (lit. i), above) results in the desired low shrinkage; however, the modulus of elasticity is then typically was raised to than 12 GPa. Any attempt to weaken the impact of the filling material onto the marginal seal under chewing stress must not result in an increase of the volume shrinkage. Towards this end, the filling material according to the invention comprises core-shell particles according to lit. ii), above. It has been surprisingly found that the incorporation of such core-shell particles into filled composite filling materials results in significantly improved performance in premolar and molar teeth.

Suitable core-shell particles according to lit. ii), above, are known as such and are disclosed e.g. in WO 2005/055961 A1 (incorporated herein by reference with respect to the composition and methods of manufacture of the core-shell polymer particles).

To the best of applicant's knowledge, core-shell polymer particles according to lit. ii), above, have never been suggested in combination with non-agglomerated nanofillers according to lit. i), above, in order to compensate for the otherwise too high modulus of elasticity in molar and premolar applications.

The dental composite material preferably has a modulus of elasticity in the hardened state in the range of 6 GPa to 10 GPa, preferably in the range of 7.0 GPa to 9.5 GPa, most preferably in the range of 7.5 GPa to 9.0 GPa.

Such overall modules of elasticity in the hardened state have been found to be particularly suitable for bulk filling restorations of molar and premolar teeth with compositions according to the present invention.

Moreover, the dental composite material exhibits a polymerization shrinkage of ≤ 3.0% by volume, preferably of ≤ 2.5% by volume, most preferably of ≤ 2.4% by volume.

As outlined above, dental composite materials with such low percentages of volume shrinkage upon polymerization were available as such in the prior art, but nevertheless were not ideally suitable for restoration of molar and premolar teeth in the bulk filling technique.

According to preferred embodiments of the present invention, the core compound of the core-shell polymer particles comprises a material selected from the group consisting of silicone; polyalkyl acrylate, preferably polybutyl acrylate; polybutadiene; styrene butadiene copolymers; and mixtures thereof.

According to yet further preferred embodiments of the present invention, the shell compound of the core-shell polymer particles comprises a material selected from the group consisting of polyalkyl methacrylate (preferably polymethyl methacrylate), optionally comprising functional groups selected from the group consisting of epoxy, carboxylic acid, amine; copolymers of polyalkyl methacrylate (preferably polymethyl methacrylate) with styrene, optionally comprising functional groups selected from the group consisting of epoxy, carboxylic acid, amine.

Currently preferred are core-shell particles comprising a polymethyl methacrylate shell and a silicone core, such as commercially available under the trademark GENIOPERL®, e.g. GENIOPERL® P22 (Wacker-Chemie GmbH, WACKER SILICONES, München (DE)).

The core-shell particles are incorporated into the dental composite material at about 0.1% by weight to about 10% by weight, preferably about 1% by weight to about 8% by weight, most preferably about 1% by weight to about 6% by weight of the core-shell polymer particles.

According to preferred embodiments of the present invention, the composite material comprises a polymerizable matrix of ethylenically unsaturated monomers, preferably selected from the group consisting of 2,2'-bis-[4-(methacryloxypropoxy)phenyl]-propane (bis-GMA); tri-(ethylene glycol) dimethacrylate (TEGDMA); urethane dimethacrylate (UDMA); diethylene glycol di(meth)acrylate; decanediol di(meth)acrylate; trimethylolpropane tri(meth)acrylate; pentaerythrite tetra(meth)acrylate; butanediol di(meth)acrylate; 1,10-decanediol di(meth)acrylate; 1,12-dodecanediol di(meth)acrylate; and mixtures thereof. Currently preferred are mixtures of TEGDMA, bis-GMA and UDMA.

In principle, the technical effect of the present invention could be used in any curing system known in the art, e.g. in hot polymerization systems, photo polymerization systems and cold/dark polymerization systems. Towards this end, compositions according to the invention may further comprise a polymerization initiator selected from the group consisting of initiators for hot polymerization; photoinitiators; and, preferably, initiators for cold polymerization. Also dual-curing systems may be configured as known in the art.

Suitable polymerization initiators for any kind of polymerization system are perfectly known to the person of routine skill in the art:

Peroxides (such as e.g. dibenzoyl peroxide) and α,α'-azobis(isobutyroethyl ester) can be used as initiators for hot polymerization.

Benzoin alkyl ethers/esters or camphorquinone can be used as initiators for photopolymerization, preferably together with a reducing agent such as e.g. an amine.

Peroxides (such as dibenzoyl peroxide or lauroyl peroxide) may be used together with amines such as e.g. N,N-diethanol-p-toluidine as initiators for cold/dark polymerization.

As used herein, "cold/dark polymerization" is understood as a polymerization that initiates and proceeds in the absence of light and under normal temperature and pressure (NTP) conditions (101'325 Pa and 20°C).

In the context of the present invention, currently preferred non-agglomerated nanofillers according to lit. i), above, are or comprise SiO₂, ZrO₂, TiO₂ or Al₂O₃, and mixtures thereof. Currently preferred are silica particles predispersed in methacrylates (e.g Nanocryl D322, Hanse Chemie AG). The non-agglomerated nanofillers are preferably present in an amount of about 0% by weight to about 15% by weight, preferably about 1% by weight to about 12% by weight, most preferably about 3% by weight to about 10% by weight, based on the total weight of the dental composite material.

Preferably the dental composite material is a cold/dark polmyerization material as defined above. The present invention is especially advantageous in the context of such cold/dark polymerization materials: Since these materials are provided as two- component compositions (base paste and catalyst paste) that immediately react upon mixing, these two components need to be sufficiently flowable in order to allow for quick and thorough mixing. In order to achieve a low shrinkage upon curing while at the same time assuring acceptable flowability, the size of the fillers had been chosen relatively big in the prior art. As has now been identified as a problem with bulk fillings especially of molar and premolar teeth, this results in an inappropriate, i.e. a too high module of elasticity. The present invention now overcomes this drawback by providing a composition defined above.

Yet another aspect of the present invention concerns the use of core-shell polymer particles with
- a core compound having a modulus of elasticity of less than about 18 MPa, preferably of less than 14 MPa, most preferably of less than about 10 MPa, and a polymethyl methacrylate shell; and
- a substantially non-elastic shell compound for the manufacture of a composite material as outlined above for dental restorations of molar and premolar teeth.

It is to be understood that conventional bondings, adhesives and primers may suitably be applied onto/into the prepared tooth prior to filling the tooth with the composite material according to the invention.

The invention will now be described by means of specific embodiments, without intending to limit the invention to these specific embodiments.

The following dental composite materials have been prepared (amounts given as weight in [g]) and investigated.

### Example 1 (comparative, not according to the invention; weight in gram):

| | | **Catalyst paste** | **Base paste** |
|---|---|---|---|
| Fillers: | Nanoparticles⁽¹⁾ | 7,0 | 7,1 |
| | Pyrogenic silicic acid⁽²⁾ | 2,8 | 2,8 |
| | Dental glass ∅0,7µm (silanised) | 10,9 | 10,9 |
| | Dental glass ∅1,2µm (silanised) | 10,9 | 10,9 |
| | Dental glass ∅7,0µm (silanised) | 55,5 | 55,0 |
| Monomers: | TEGDMA | 4,33 | 4,39 |
| | bis-GMA | 4,64 | 4,74 |
| | UDMA | 3,60 | 3,64 |
| Initiators: | Dibenzoyl peroxide | 0,4 | - |
| | Butylated hydroxytoluene⁽³⁾ | 0,05 | - |
| | N,N-diethanol-p-toluidine | - | 0,748 |
| Sum: | | 100,12 | 100,218 |

| | | | |
|---|---|---|---|
| ⁽¹⁾: Nanocryl D322 (SiO₂ Particles 20nm), Hanse Chemie AG. The nanoparticles are pre-dispersed in TEGDMA and UDMA in the commercially available product Nanocryl D322. The nanoparticles of Nanocryl D322 are listed separately in the above table as fillers, while the TEGDMA and UDMA are listed as monomers. ⁽²⁾: Aerosil R812S, Degussa Evonik ⁽³⁾: Inhibitor, Stabilizer | | | |

The following preparation and test regimen has been used in all examples:
The catalyst and base paste have been conventionally mixed and the quantitative analysis of the marginal adaptation was determined after a chewing simulation test as outlined in Göhring et al., American Journal of Dentistry, Vol. 16, No. 4 (2003), p. 275-282 (mixed class II cavity). Parabond (Coltène/Whaledent AG) has been used as a primer/adhesive, according to the manufacturer's instructions. The flexural modulus has been determined according to ISO4049:2000.

The hardened composite of example 1 had the following properties:

| **Modulus of elasticity** | **Volume shrinkage** | **Continuous margin** |
|---|---|---|
| 16'000 MPa | 2.1% | 60% |

### Example 2 (according to the invention; weight in gram):

| | | **Catalyst paste** | **Base paste** |
|---|---|---|---|
| Fillers: | Nanoparticles⁽¹⁾ | 8,06 | 7,86 |
| | Pyrogenic silicic acid⁽²⁾ | 2,8 | 2,8 |
| | Dental glass ∅0,7µm (silanised) | 9.7 | 9,8 |
| | Dental glass ∅1,2µm (silanised) | 9.7 | 9,8 |
| | Dental glass ∅7,0µm (silanised) | 49,8 | 50,1 |
| | Genioperl® P22 (Wacker) | 4,9 | 5,0 |
| Monomers: | TEGDMA | 5,0 | 4,86 |
| | bis-GMA | 5,37 | 5,24 |
| | UDMA | 4,16 | 4,04 |
| Initiators: | Dibenzoyl peroxide | 0,46 | - |
| | Butylated hydroxytoluene⁽³⁾ | 0,05 | |
| | N,N-diethanol-p-toluidine | - | 0,681 |
| Sum: | | 100 | 100,181 |

| | | | |
|---|---|---|---|
| ⁽¹⁾: Nanocryl D322 (SiO₂ Particles 20nm), Hanse Chemie AG. The nanoparticles are pre-dispersed in TEGDMA and UDMA in the commercially available product Nanocryl D322. The nanoparticles of Nanocryl D322 are listed separately in the above table as fillers, while the TEGDMA and UDMA are listed as monomers. ⁽²⁾: Aerosil R812S, Degussa Evonik ⁽³⁾: Inhibitor, Stabilizer | | | |

The hardened composite had the following properties:

| **Modulus of elasticity** | **Volume shrinkage** | **Continuous margin** |
|---|---|---|
| 9'000 MPa | 2.4% | 79% |

From a comparison of the results of examples 1 and 2, it is evident that the incorporation of the core-shell particles results in a strikingly reduced modulus of elasticity, thereby rendering the composition suitable for restoring molar and premolar teeth with the bulk filling technique; the volume shrinkage is acceptable, the continuous margin even improved.

## Claims

1. Hardenable dental composite material, comprising
i) non-agglomerated nanofillers having particle sizes of about 1 to about 50 nm;
ii) core-shell polymer particles with
- an elastic core compound, preferably having a modulus of elasticity of less than about 18 MPa, more preferably of less than 14 MPa, most preferably of less than about 10 MPa; and
- a substantially non-elastic shell compound.

2. Dental composite material according to claim 1, having a modulus of elasticity in the hardened state in the range of 6 GPa to 10 GPa, preferably in the range of 7.0 GPa to 9.5 GPa, most preferably in the range of 7.5 GPa to 9.0 GPa.

3. Dental composite material according to one of claims 1 or 2, having a polymerization shrinkage of ≤ 3.0% by volume, preferably of ≤ 2.5% by volume, most preferably of ≤ 2.4% by volume.

4. Dental composite material according to one of claims 1 to 3, wherein the core compound of the core-shell polymer particles comprises a material selected from the group consisting of silicone; polyalkyl acrylate, preferably polybutyl acrylate; polybutadiene; styrene butadiene copolymers; and mixtures thereof.

5. Dental composite material according to one of claims 1 to 4, wherein the shell compound of the core-shell polymer particles comprises a material selected from the group consisting of polyalkyl methacrylate (preferably polymethyl methacrylate), optionally comprising functional groups selected from the group consisting of epoxy, carboxylic acid, amine; copolymers of polyalkyl methacrylate (preferably polymethyl methacrylate) with styrene, optionally comprising functional groups selected from the group consisting of epoxy, carboxylic acid, amine.

6. Dental composite material according to one of claims 1 to 5, comprising about 0.1% by weight to about 10% by weight, preferably about 1% by weight to about 8% by weight, most preferably about 1% by weight to about 6% by weight of the core-shell polymer particles.

7. Dental composite material according to one of claims 1 to 6, further comprising ethylenically unsaturated monomers, preferably selected from the group consisting of 2,2'-bis-[4-(methacryloxypropoxy)-phenyl]-propane (bis-GMA); tri-(ethylene glycol) dimethacrylate (TEGDMA); urethane dimethacrylate (UDMA); diethylene glycol di(meth)acrylate; decanediol di(meth)acrylate; trimethylolpropane tri(meth)acrylate; pentaerythrite tetra(meth)acrylate; butanediol di(meth)acrylate; 1,10-decanediol di(meth)acrylate; 1,12-dodecanediol di(meth)acrylate; and mixtures thereof.

8. Dental composite material according to claim 7, further comprising a polymerization initiator selected from the group consisting of initiators for hot polymerization; photoinitiators; and, preferably, initiators for cold polymerization.

9. Dental composite material according to one of claims 1 to 8, wherein the non-agglomerated nanofiller is or comprises SiO₂, ZrO₂, TiO₂ or Al₂O₃.

10. Dental composite material according to one of claims 1 to 9, wherein the dental composite material is hardenable at room temperature, ambient pressure and in the dark.

11. Dental composite material according to one of claims 1 to 10, constituted by mixing a base paste and a catalyst paste.

12. Use of core-shell polymer particles with
- a core compound having a modulus of elasticity of less than about 18 MPa, preferably of less than 14 MPa, most preferably of less than about 10 MPa, and a polymethyl methacrylate shell; and
- a substantially non-elastic shell compound
for the manufacture of a composite material according to one of claims 1 to 11 for dental restorations of molar and premolar teeth.
